Europäisches Patentamt

European Patent Office

Office européen des brevets

(19)

(11) Veröffentlichungsnummer: **0 071 770**
A2

(12) # EUROPÄISCHE PATENTANMELDUNG

(21) Anmeldenummer: 82106116.5

(22) Anmeldetag: 08.07.82

(51) Int. Cl.³: **B 01 J 23/76,** B 01 J 23/56,
B 01 J 23/74, B 01 J 23/40,
C 07 C 1/02, C 07 C 11/00

(30) Priorität: 05.08.81 DE 3130988

(43) Veröffentlichungstag der Anmeldung: 16.02.83
Patentblatt 83/7

(84) Benannte Vertragsstaaten: **BE DE FR GB IT NL**

(71) Anmelder: **SCHERING AKTIENGESELLSCHAFT Berlin
und Bergkamen, Waldstrasse 14 Postfach 1540,
D-4619 Bergkamen (DE)**

(72) Erfinder: **Gökcebay, Haluk, Dipl.-Chem.Dr.-Ing.,
Ringstrasse 33, D-7500 Karlsruhe 41 (DE)**
Erfinder: **Schulz, Hans, Prof.Dr.,
Insterburgerstrasse 11 d, D-7500 Karlsruhe (DE)**

(54) **Katalysator und Verfahren zur Herstellung von Olefinen, insbesondere linearen alpha-Olefinen, aus Synthesegas.**

(57) Die Erfindung betrifft ein Verfahren zur Herstellung von bevorzugt lineraren $\alpha$-Olefinen aus Synthesegas sowie die Katalysatoren hierfür. Die Katalysatoren sind Matrix-Katalysatoren, bei denen die aktive Komponente, insbesondere Eisen, Kobalt, Nickel oder Ruthenium, eingebunden in einer oxydischen Matrix-Überschuss-Komponente.

EP 0 071 770 A2

Katalaysator und Verfahren zur Herstellung von
Olefinen - insbesondere linearen α-Olefinen - aus
Synthesegas

Die Erfindung betrifft neue Katalysatoren, deren aktive
Komponente in einer inaktiven Matrix-Komponente vorliegt, sowie ein Verfahren zur Herstellung von Olefinen,
insbesondere linearen α-Olefinen, aus Synthesegas.

Bei den Katalysatoren der bekannten technischen Synthesen
ist Eisen allgemein die Hauptkomponente. Beim Festbettverfahren der Sasol beträgt der Anteil der α-Olefine an den
n-Olefinen nur 60 % (H. Pichler, H. Schulz, D. Kühne,
Brennstoff-Chemie, 49, 344 (1968)), wobei der Olefinanteil wiederum knapp 50 % der Kohlenwasserstoffe (Bereich
$C_6$ bis $C_{12}$) ausmacht. Beim Flugstaubverfahren erreicht der
Anteil der α-Olefine an den n-Olefinen 70 bis 75 %, wobei
der Olefinanteil der Kohlenwasserstoffe auch 70 bis 75
Mol-% beträgt. Beim Flugstaubverfahren haben die erhaltenen Olefine jedoch zu erheblichen Anteilen ein verzweigtes
Kohlenstoffgerüst (Verzweigungsgrad etwa 50 tertiäre C-
Atome pro 1000 C-Atome), so daß der Anteil der linearen
Olefine entsprechend erniedrigt ist. Außer dieser Ausbeuterniedrigung ist auch die Auftrennung komplexer Gemische isomerer Olefine ein fast unlösbares technisches
Problem.

In der Offenlegungsschrift DE-OS 25 07 647 wird ein
Katalysator für die Herstellung niedriger Olefine ($C_2/C_4$)
aus Synthesegas beschrieben: Der Katalysator wird durch

0071770

Fällung mit Ammoniak hergestellt und durch Formierung mit Kohlenoxid vorbehandelt, d. h. mit Kohlenstoff teilweise belegt, um eine gute Aktivität und Selektivität zu erreichen. Als Produkte werden bevorzugt niedrige Kohlenwasserstoffe ($C_2C_4$-Olefine) und Sauerstoffverbindungen erzeugt. Es handelt sich dabei um "Mangan-Katalysatoren"; Mangan selber ergibt dort eine Syntheseaktivität.

In der Offenlegungsschrift DE-OS 25 18 964 bzw. DE-OS 25 36 488 werden Katalysatoren mit den Komponenten Eisen-Mangan und Eisen-Vanadium bzw. Eisen-Titan beschrieben. Dabei soll der Mangananteil bzw. Vanadiumanteil höchstens dem Gewichtsanteil des Eisens entsprechen. Das Gewichtsverhältnis Titan zu Eisen soll 1 : 2 bis 1 : 10 betragen. Produktionsziel ist wiederum die Herstellung niedriger Olefine.

Dagegen ist es Aufgabe der Erfindung, Katalysatoren sowie ein Verfahren unter Verwendung dieser Katalysatoren zu schaffen, womit man aus Synthesegas mit guter Selektivität die als Chemierohstoff bedeutsamen geradkettigen Olefine mit endständiger Doppelbindung erhält.

Die Aufgabe wird gelöst durch einen Katalysator, der dadurch gekennzeichnet ist, daß die aktive Katalysatorkomponente eingebunden in einer oxidischen Matrixüberschußkomponente vorliegt.

Die aktive Komponente ist dabei vorzugsweise Co, Ni, Ru und insbesondere Fe. Die Matrix ist vorzugsweise gebildet aus Oxiden von Titan, Mangan oder Vanadin.

Das Massenverhältnis von Matrixkomponente zu aktiver Komponente ist vorzugsweise 3 bis 18, insbesondere 4 bis 14.

Durch Alkalisierung, vorzugsweise mit Kaliumcarbonat, im Bereich von 1 bis 8 Gew.-%, wird ein bevorzugter Katalysator erhalten.

Weiterhin wird ein bevorzugter, aktivierter Katalysator durch reduzierende Vorbehandlung mit Wasserstoff bei 350 °C bis 450 °C erhalten.

Gegenstand der Erfindung ist weiterhin ein Verfahren zur Herstellung bevorzugt linearer $\alpha$-Olefine aus Synthesegas, dadurch gekennzeichnet, daß man die genannten Katalysatoren im Temperaturbereich von 170 ° bis 320 °C und im Druckbereich von 1 bis 150 bar mit Synthesegas beaufschlagt.

Das besondere Merkmal der erfindungsgemäßen Katalysatoren besteht, wie ausgeführt, darin, daß die aktive Komponente in feiner Verteilung in eine Matrixkomponente mit geeigneten Eigenschaften eingebettet ist.

Im Falle der Fischer-Tropsch-Synthese führt die Einbettung der aktiven Komponente in eine als Matrix geeignete Oxid-Komponente zur Desaktivierung der unselektiven Hydrierzentren, während die Fischer-Tropsch-Synthesezentren, an denen der Molekülkettenaufbau verläuft, in aktiver Form verstärkt auftreten. Dabei liegt ein Katalysatorsystem vor, in dem eine selektive $\alpha$-Olefinsynthese verläuft, während die in bekannten Systemen übliche Begleitreaktion der Olefindoppelbindungsverschiebung vollständig unterdrückt werden kann (Fig. 1). Auch die Begleitreaktion der Hydrierung der synthetisierten Olefine ist weitgehend zurückgedrängt (Fig. 2).

Als Matrix-Komponenten haben Titanoxid, Manganoxid sowie auch Vanadiumoxid gute Eigenschaften. Sie besitzen eine reaktionslenkende Wirkung in Richtung auf eine hohe Selektivität für $\alpha$-Olefine (Fig. 1 und 2). Die Verdünnung mit einer nicht matrix-wirksamen Komponente (Aerosil) ergibt dagegen keine befriedigende Produktselektivität (Vergleichsbeispiel B). Die Matrix-Komponente wird in erheblichem Überschuß angewendet. Der Bereich typischer Massenverhältnisse in den Matrix-Katalysatoren, angegeben als Masse Titan, Vanadium oder Mangan bezogen auf die Masse an aktiver Komponente, liegt im Bereich von 3 bis 18 Masseteilen, vorzugsweise 4 bis 14 Masseteilen, (Synthese-Beispiele 1 bis 16).

Die Matrix-Komponenten selber haben keine bzw. nur eine geringfügige Aktivität für die Umsetzung von Synthesegas (Vergleichsbeispiele C und D) hauptsächlich zu Methan.

Verschiedene Nebenbestandteile, wie z. B. ZnO, können die Aktivität der Katalysatoren verbessern. Ein wichtiger aktivierender Zusatz der Matrix-Katalysatoren ist Alkali, das bevorzugt als $K_2CO_3$ eingebracht wird. Günstig ist eine relativ starke Alkalisierung von beispielsweise 3 bis 5 Gew.-% $K_2O$ bezogen auf die Masse des getrockneten, durch Fällung hergestellten Katalysators. Als strukturverbessernde Katalysatorkomponenten sind $Al_2O_3$ und Aerosil geeignet.

Die Katalysatorherstellung erfolgt bevorzugt durch Fällung (Beispiel 2) der Komponenten aus den Salzlösungen. Es können jedoch auch frisch gefällte Niederschläge (Oxidhydrate, basische karbonathaltige Niederschläge) miteinander homogen vermischt werden (Beispiel 1). Die gefällten Niederschläge werden in üblicher Weise getrocknet und bei etwa 300 °C getempert. Auch eine homogene Mischung der fein gemahlenen Oxide (gemeinsam mit Promotoren) und nachfolgende Temperaturbehandlung der Mischung kann zu aktiven Matrix-Katalysatoren führen.

Die Selektivität für einzelne Produktgruppen läßt sich weiterhin in üblicher Weise durch geeignete Wahl der Verfahrensbedingungen steuern. Die geeigneten Reaktionstemperaturen liegen zwischen 170 ° und 340 °C. Mit Nickel, Kobalt und Ruthenium als aktiven Komponenten liegt der Arbeitsbereich bevorzugt zwischen 180 und 280 °C, mit Eisen bevorzugt zwischen 210 und 320 °C.

Der Reaktionsdruck kann in einem weiten Bereich zur Optimierung der Produktselektivität angepaßt werden.

Mit Eisen und Ruthenium liegt der Reaktionsdruck bevorzugt zwischen 1 und 120 bar, mit Nickel im Bereich von 1 bis 4 bar und mit Kobalt im Bereich von 1 bis 80 bar.

Das Synthesegas wird üblicherweise mit einem $H_2/CO$-Verhältnis von 2 : 1 verwendet. Je nach Katalysatorsystem erstreckt sich der Bereich geeigneter $H_2/CO$-Verhältnisse jedoch von $H_2/CO$ = 0.3 bis 4.

Die Raumgeschwindigkeit liegt üblicherweise im Bereich der Werte 100 bis 1000 Liter Synthesegas (bezogen auf 0 °C und 1 bar) pro Stunde und pro 1 Liter Reaktionsraum).

Beispiel 1

Herstellung eines Eisen-Titanoxid-Matrix-Fällungskatalysators.

Ausgehend von TiCl$_4$ wird zunächst durch Hydrolyse das kolloid disperse Titanoxid-hydrat erhalten. Daraus wird durch Umsetzung mit Kaliumkarbonat das Titankarbonat hergestellt. Der Katalysator wird durch Vermischen des frisch gefällten Titankarbonats mit einem aus Eisennitrat getrennt hergestellten Karbonatniederschlag erhalten. Die Trocknung der Mischung wird bei 110 °C vorgenommen.

Beispiel 2

Herstellung eines Eisen-Manganoxid-Matrix-Fällungskatalysators.

Ausgehend von der Nitratlösung von Eisen und Mangan wird in der Siedehitze die gemeinsame Fällung der Karbonate vorgenommen. Der nitratfrei gewaschene Niederschlag wird bei 110 °C getrocknet.

Beispiel 3

Herstellung des Fe-ZnO-V$_2$O$_5$-Katalysators.

Ammonvanadat wurde in Wasser bei 70 °C aufgelöst und in eine Lösung von Eisen(III)- und Zinknitrat gegeben. Der pH-Wert der Lösung wurde anschließend mit K$_2$CO$_3$ auf 6.8 eingestellt.

Beispiel 4

Herstellung des Fe-V$_2$O$_5$-Katalysators.

Ammonvanadat wurde in Wasser bei 70 °C aufgelöst und in eine Lösung von Eisen(III)-Nitrat gegeben. Der pH-Wert der Lösung wurde anschließend mit K$_2$CO$_3$ auf 6.8 eingestellt.

Beispiel 5

Herstellung eines Kobalt-Manganoxid-Matrix-Fällungskatalysators.

Ausgehend von der Nitratlösung von Kobalt und Mangan wird in der Siedehitze die gemeinsame Fällung der Karbonate vorgenommen. Der nitratfrei gewaschene Niederschlag wird bei 110 °C getrocknet.

Beispiel 6

Herstellung eines Nickel-Manganoxid-Matrix-Fällungskatalysators.
Ausgehend von der Nitratlösung von Nickel und Mangan wird in der Siedehitze die gemeinsame Fällung der Karbonate vorgenommen. Der nitratfrei gewaschene Niederschlag wird bei 110 °C getrocknet.

Beispiel 7

Herstellung eines Ruthenium-Manganoxid-Matrix-Fällungskatalysators.
Die Ruthenium- und Mangankomponenten werden getrennt als aktive Niederschläge hergestellt und homogen vermischt.

Aus der alkalischen Ruthenatlösung wird aktives $RuO_2$ durch reduktive Fällung mit Methanol erhalten. Aktives Mangancarbonat wird aus Mangannitratlösung durch Fällung mit $K_2CO_3$-Lösung gewonnen. Die Niederschläge werden - wie erwähnt - homogen vermischt.

Synthesebeispiele

Die Synthesebeispiele 1 bis 16 (siehe Tabelle) zeigen die erfindungsgemäßen Ergebnisse mit den Eisen-Titanoxid-, Eisen-Manganoxid- und Eisen-Vanadiumoxid-Matrixkatalysa-

0071770

toren im Vergleich zu einem nicht modifizierten Eisenkatalysator (Vergleichsbeispiel A), zu Titanoxid und Manganoxid ohne Eisen als Katalysatorkomponente (Vergleichsbeispiel C und D) und zu einem mit Aerosil verdünnten Eisenkatalysator (Vergleichsbeispiel B).


Beispiele für die Aktivierung der Katalysatoren


a) Fe/Mn-Matrix-Katalysator:

Nach Aufheizen des Katalysators im Stickstoffstrom wird der Katalysator bei 380 °C und 1 bar mit einem Kohlenmonoxid-Wasserstoff-Gemisch (1 : 1) bei einer Raumgeschwindigkeit von 1500 (Liter Gas/Liter Katalysator . h) während 24 Stunden aktiviert.


b) Fe/Ti- bzw. Fe/V-Matrix-Katalysator:

Wie a), jedoch erfolgt die Aktivierung nur mit $H_2$ bei 370 °C, Raumgeschwindigkeit 2000.


c) Co/Mn:

Wie b), jedoch bei einer Temperatur von 350 °C.


d) Ni/Mn:

Wie b), jedoch bei einer Temperatur von 350 °C.


e) Ru/Mn:

Wie b), jedoch bei einer Temperatur von 200 °C.

0071770

Zusammensetzung (in Massenteilen) der in den Synthesebeispielen bzw. Vergleichsbeispielen eingesetzten
Katalysatoren

Katalysator 1:   100 Fe (3.0 $K_2O$)
              2:   100 Fe : 800 Ti (4.8 $K_2O$)
              3:   100 Fe : 1200 Ti (2.3 $K_2O$)
              4:   100 Fe : 1200 Mn (5.3 $K_2O$)
              5:   100 Fe : 600 Mn (2.6 $K_2O$)
              6:   100 Fe : 300 Mn (2.0 $K_2O$)
              7:   100 Fe : 600 Mn (0 $K_2O$)
              8:   100 Fe : 1200 Aerosil (3.1 $K_2O$)
              9:   100 Mn (3.2 $K_2O$)
             10:   100 Ti (2.8 $K_2O$)
             11:   100 Fe : 800 Mn (3.8 $K_2O$)
             12:   100 Fe : 200 Zn 600 V (2.5 $K_2O$)
             13:   100 Fe : 600 V

Die Katalysatoren enthalten als zusätzliche Bestandteile
ca. 7 Massenprozent an Aerosil und ca. 1 Massenprozent an
$Al_2O_3$, bezogen auf die Summe der Metallkomponenten. Der
Alkalianteil (als $K_2O$) ist ebenfalls bezogen auf die Summe
der Metallkomponenten.

**Fig. 1:** α-Olefinanteile der n-Olefine in Abhängigkeit von der C-Zahl

**Fig. 2:** Olefinanteile der Kohlenwasserstoffe in Abhängigkeit von der C-Zahl

<u>Tabelle:</u> Beispiele für Syntheseergebnisse mit den neuen Katalysatoren

| Synthesebeispiel | 1 | 2 | 3 | 4 | 5 | 6 |
|---|---|---|---|---|---|---|
| Katalysator | 2 | 3 | 2 | 4 | 5 | 6 |
| Katalysatorzusammensetzg. | 1Fe/8Ti | 1Fe/12Ti | 1Fe/8Ti | 1Fe/12Mn | 1Fe/6Mn | 1Fe/3Mn |

<u>Selektivität in C-%</u>

| | 1 | 2 | 3 | 4 | 5 | 6 |
|---|---|---|---|---|---|---|
| $\alpha$-Olefinanteil der n-Olefine $C_4/C_9$ | 96/93 | 98/92 | 97/93 | 98/97 | 98/97 | 85/65 |
| $C_{10} - C_{14}$ | 75 | 54 | 85 | 96 | 60 | 55 |
| $C_{15} +$ | 52 | 40 | 75 | 92 | 42 | 42 |
| Olefinanteil in den Fraktionen $C_2/C_4/C_9$ | 74/79/75 | 61/75/52 | 85/86/82 | 86/84/81 | 77/85/70 | 38/76/58 |
| $C_{10} - C_{14}$ | 65 | 50 | 80 | 80 | 65 | 53 |
| $C_{15} +$ | 52 | 44 | 76 | 78 | 50 | 50 |
| Anteil linearer Aliphaten in $C_4/C_8$ | 93/78 | 95/89 | 95/81 | 94/89 | 98/89 | 95/90 |

Tabelle: Beispiele für Syntheseergebnisse mit den neuen Katalysatoren (Fortsetzung)

| Synthesebeispiel | 1 | 2 | 3 | 4 | 5 | 6 |
|---|---|---|---|---|---|---|
| **Produktverteilung** | | | | | | |
| $C_1$ | 11 | 13 | 12 | 6 | 9 | 14 |
| $C_2/C_3$ | 10/12 | 11/12 | 11/13 | 7/10 | 8/11 | 9/13 |
| $C_4/C_5/C_6$ | 10/8/7 | 10/8/7 | 8/7/6 | 9/8/7 | 9/8/7 | 11/9/8 |
| $C_7/C_9$ | 16 | 15 | 16 | 25 | 20 | 15 |
| $C_{10}/C_{14}$ | 15 | 11 | 15 | 19 | 19 | 14 |
| $C_{15}+$ | 5 | 5 | 7 | 7 | 6 | 4 |
| Alkohole | 6 | 8 | 5 | 2 | 3 | 3 |
| **Synthesebedingungen** | | | | | | |
| Temperatur, °C | 250 | 250 | 250 | 250 | 250 | 250 |
| Druck, bar | 10 | 10 | 10 | 10 | 10 | 10 |
| $H_2/CO$ im Synthesegas | 2 | 2 | 1 | 2 | 2 | 2 |
| Raumgeschwindigkeit, 1/1 h | 180 | 200 | 180 | 180 | 180 | 190 |
| $(H_2+CO)$-Umsatz, % | 28 | 34 | 15 | 30 | 36 | 60 |

Tabelle: Beispiele für Syntheseergebnisse mit den neuen Katalysatoren (Fortsetzung)

| Synthesebeispiel | 7 | 8 | 9 | 10 | 11 | 12 | 13 |
|---|---|---|---|---|---|---|---|
| Katalysator | 5 | 4 | 4 | 11 | 11 | 12 | 13 |
| Katalysatorzusammensetzg. | 1Fe/6Mn | 1Fe/12Mn | 1Fe/12 Mn | 1Fe/8Mn | 1Fe/8Mn | 1Fe/2Zn/6V | 1Fe/6V |

Selektivität in C-%

| | 7 | 8 | 9 | 10 | 11 | 12 | 13 |
|---|---|---|---|---|---|---|---|
| α-Olefinanteil der n-Olefine $C_4/C_9$ | 97/97 | 95/94 | 80/63 | 95/93 | 92/91 | 95/94 | 92/75 |
| $C_{10} - C_{14}$ | 83 | 90 | 40 | 93 | 77 | 90 | 40 |
| $C_{15}+$ | 60 | 87 | 40 | 91 | 77 | 88 | 30 |
| Olefinanteil in den Fraktionen $C_2/C_4/C_9$ | 84/87/83 | 72/69/70 | 49/82/67 | 69/64/56 | 70/72/65 | 85/89/86 | 67/80/65 |
| $C_{10} - C_{14}$ | 78 | 57 | 58 | 53 | 63 | 81 | 45 |
| $C_{15}+$ | 68 | 42 | 42 | 40 | 62 | 78 | 30 |
| Anteil linearer Aliphaten in $C_4/C_8$ | 98/89 | 94/90 | 98/81 | 96/90 | 92/88 | 98/81 | 93/79 |

Tabelle: Beispiele für Syntheseergebnisse mit den neuen Katalysatoren (Fortsetzung)

| Synthesebeispiel | 7 | 8 | 9 | 10 | 11 | 12 | 13 |
|---|---|---|---|---|---|---|---|
| **Produktverteilung** | | | | | | | |
| $C_1$ | 8 | 9 | 13 | 7 | 19 | 16 | 13 |
| $C_2/C_3$ | 8/10 | 12/13 | 10/13 | 9/14 | 13/14 | 14/16 | 11/13 |
| $C_4/C_5/C_6$ | 9/9/8 | 10/9/8 | 11/10/9 | 13/10/8 | 11/8/5 | 13/9/7 | 11/9/7 |
| $C_7/C_9$ | 21 | 14 | 18 | 16 | 11 | 11 | 14 |
| $C_{10}/C_{14}$ | 20 | 11 | 10 | 11 | 6 | 7 | 8 |
| $C_{15}+$ | 5 | 7 | 3 | 3 | 3 | 2 | 6 |
| Alkohole | 2 | 7 | 3 | 9 | 10 | 5 | 8 |
| **Synthesebedingungen** | | | | | | | |
| Temperatur, $^{\circ}C$ | 250 | 250 | 280 | 220 | 250 | 280 | 250 |
| Druck, bar | 10 | 60 | 10 | 90 | 30 | 10 | 10 |
| $H_2/CO$ im Synthesegas | 1 | 2 | 2 | 2 | 2 | 2 | 2 |
| Raumgeschwindigkeit, 1/1 h | 180 | 100 | 200 | 100 | 100 | 300 | 150 |
| $(H_2+CO)$-Umsatz, % | 15 | 60 | 70 | 74 | 42 | 55 | 40 |

0071770

Tabelle: Beispiele für Syntheseergebnisse mit den neuen Katalysatoren

| Synthesebeispiel | 14 | 15 | 16 |
|---|---|---|---|
| Katalysator | 14 | 15 | 16 |
| Katalysatorzusammensetzg. | 1 Co/6 Mn | 1 Ni/6 Mn | 1 Ru/10 Mn |

Selektivität in C-%

| | | | |
|---|---|---|---|
| $\alpha$-Olefinanteil der n-Olefine $C_4/C_9$ | 72/64 | 68/60 | 64/50 |
| $C_{10} - C_{14}$ | 60 | 56 | 45 |
| $C_{15}+$ | 55 | 53 | 41 |
| Olefinanteil in den Fraktionen $C_2/C_4/C_9$ | 68/72/63 | 60/65/59 | 40/65/58 |
| $C_{10} - C_{14}$ | 59 | 54 | 52 |
| $C_{15}+$ | 54 | 51 | 49 |
| Anteil linearer Aliphaten in $C_4/C_8$ | 96/80 | 85/78 | 97/91 |

Tabelle: Beispiele für Syntheseergebnisse mit den neuen Katalysatoren (Fortsetzung)

| Synthesebeispiel | 14 | 15 | 16 |
|---|---|---|---|
| **Produktverteilung** | | | |
| $C_1$ | 18 | 19 | 32 |
| $C_2/C_3$ | 5/10 | 6/12 | 4/8 |
| $C_4/C_5/C_6$ | 12/8/7 | 13/9/8 | 8/8/7 |
| $C_7/C_9$ | 13 | 13 | 12 |
| $C_{10}/C_{14}$ | 9 | 9 | 10 |
| $C_{15}+$ | 13 | 9 | 9 |
| Alkohole | 5 | 2 | 2 |
| **Synthesebedingungen** | | | |
| Temperatur, $^oC$ | 200 | 190 | 180 |
| Druck, bar | 20 | 2 | 90 |
| $H_2/CO$ im Synthesegas | 2 | 2 | 2 |
| Raumgeschwindigkeit, 1/1 h | 100 | 100 | 100 |
| $(H_2+CO)$-Umsatz, % | 45 | 30 | 43 |

0071770

<u>Tabelle:</u> Vergleichsbeispiele

| Vergleichsbeispiel | A | B [1) | C | D |
|---|---|---|---|---|
| Katalysator | 1. | 8 | 9 | 10 |
| Katalysatorzusammensetzg. | Fe | 1Fe/12Ae | Mn | Ti |

<u>Selektivität in C-%</u>

| | A | B | C | D |
|---|---|---|---|---|
| $\alpha$-Olefinanteil der n-Olefine $C_4/C_9$ | 49/13 | 44/- | 85/- | -/- |
| $C_{10} - C_{14}$ | 13 | - | - | - |
| $C_{15} +$ | 13 | - | - | - |
| Olefinanteil in den Fraktionen $C_2/C_4/C_9$ | 15/58/40 | 15/57/- | 83/73/- | -/-/- |
| $C_{10} - C_{14}$ | 30 | - | - | - |
| $C_{15} +$ | 22 | - | - | - |
| Anteil linearer Aliphaten in $C_4/C_8$ | 97/87 | 97/- | -/- | -/- |

<u>**Tabelle:**</u> Vergleichsbeispiele (Fortsetzung)

| Vergleichsbeispiel | A | B [1] | C | D |
|---|---|---|---|---|
| **Produktverteilung** | | | | |
| $C_1$ | 21 | 52 | 65 | 98 |
| $C_2/C_3$ | 13/18 | 19/17 | 12/10 | 2/– |
| $C_4/C_5/C_6$ | 12/9/7 | 8/3/1 | 6/4/2 | –/–/– |
| $C_7/C_9$ | 12 | – | 1 | – |
| $C_{10}/C_{14}$ | 5 | – | – | – |
| $C_{15}{}^+$ | 1 | – | – | – |
| Alkohole | 2 | – | – | – |
| <u>**Synthesebedingungen**</u> | | | | |
| Temperatur, °C | 250 | 250 | 340 | 340 |
| Druck, bar | 10 | 10 | 10 | 10 |
| $H_2/CO$ im Synthesegas | 2 | 2 | 2 | 2 |
| Raumgeschwindigkeit, 1/1 h | 200 | 180 | 180 | 180 |
| $(H_2+CO)$-Umsatz, % | 62 | 8 | 6 | 2 |

1) Katalysator enthält als Promotor nur das Aerosil (Fa. Degussa)

Patentansprüche

1. Katalysator zur Herstellung von bevorzugt linearen $\alpha$-Olefinen aus Synthesegas, dadurch gekennzeichnet, daß die aktive Katalysatorkomponente eingebunden in einer oxidischen Matrixüberschußkomponente vorliegt.

2. Katalysator nach Anspruch 1, dadurch gekennzeichnet, daß die aktive Komponente Fe, Co, Ni oder Ru ist.

3. Katalysatoren nach Anspruch 1 und 2, dadurch gekennzeichnet, daß die aktive Komponente Eisen ist.

4. Katalysator nach den Ansprüchen 1, 2 und 3, dadurch gekennzeichnet, daß die Matrix aus Oxiden von Titan, Mangan oder Vanadin gebildet ist.

5. Katalysator nach den Ansprüchen 1 bis 4, dadurch gekennzeichnet, daß die Matrixkomponente ein Massenverhältnis von 3 bis 18, bevorzugt 4 bis 14, bezogen auf die aktive Komponente, aufweist.

6. Katalysator nach den Ansprüchen 1 bis 5, dadurch gekennzeichnet, daß eine Alkalisierung, vorzugsweise mit Kaliumkarbonat, im Bereich von 1 bis 8 Massenprozent, bezogen auf den getrockneten Katalysator, vorliegt.

7. Katalysator nach den Ansprüchen 1 bis 6, dadurch gekennzeichnet, daß er durch eine reduzierende Vorbehandlung im Bereich zwischen 350 und 450 °C mit Wasserstoff aktiviert worden ist.

8. Verfahren zur Herstellung bevorzugt linearer $\alpha$-Olefine aus Synthesegas, dadurch gekennzeichnet, daß man die Katalysatoren gemäß den Ansprüchen 1 bis 7 im Temperaturbereich von 170 ° bis 340 °C und im Druckbereich von 1 bis 150 bar mit Synthesegas beaufschlagt.